(19) Europäisches Patentamt / European Patent Office / Office européen des brevets

(11) **EP 3 116 552 B1**

(12) **EUROPÄISCHE PATENTSCHRIFT**

(45) Veröffentlichungstag und Bekanntmachung des Hinweises auf die Patenterteilung:
01.07.2026   Patentblatt 2026/27

(21) Anmeldenummer: **15708210.8**

(22) Anmeldetag: **06.03.2015**

(51) Internationale Patentklassifikation (IPC):
*A61L 2/10* (2026.01)    *B01J 3/04* (2006.01)
*C02F 1/32* (2023.01)

(52) Gemeinsame Patentklassifikation (CPC):
**A61L 2/10**

(86) Internationale Anmeldenummer:
PCT/EP2015/054698

(87) Internationale Veröffentlichungsnummer:
WO 2015/135844 (17.09.2015 Gazette 2015/37)

(54) **VERFAHREN ZUR KONTINUIERLICHEN VIRUSINAKTIVIERUNG**

METHOD FOR CONTINUOUS VIRAL INACTIVATION

PROCÉDÉ D'INACTIVATION DE VIRUS EN CONTINU

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität:  11.03.2014  EP 14158845

(43) Veröffentlichungstag der Anmeldung:
18.01.2017   Patentblatt 2017/03

(73) Patentinhaber: **EMD Millipore Corporation**
**Burlington, MA 01803 (US)**

(72) Erfinder:
• **LOBEDANN, Martin**
 **51069 Köln (DE)**
• **KLUTZ, Stephan**
 **51379 Leverkusen (DE)**

• **KURT, Safa Kutup**
 **44135 Dortmund (DE)**

(74) Vertreter: **Merck Patent Association**
**Merck Patent GmbH**
**64271 Darmstadt (DE)**

(56) Entgegenhaltungen:
EP-A1- 0 378 716     WO-A2-98/26236
US-A- 2 530 648     US-A1- 2006 162 912

• **SAXENA A K ET AL: "Coiled Configuration for Flow Inversion and Its Effect on Residence Time Distribution", AICHE JOURNAL, JOHN WILEY & SONS, INC, US, vol. 30, no. 3, 1 May 1984 (1984-05-01), pages 363 - 368, XP002505094, ISSN: 0001-1541, DOI: 10.1002/AIC.690300303**

**Beschreibung**

Die vorliegende Erfindung betrifft ein Verfahren zur kontinuierlichen Virusinaktivierung. Im Besonderen betrifft die Erfindung eine Vorrichtung und ein Verfahren zur kontinuierlichen Virusinaktivierung in einer Verweilzeitschleife bei niedrigem pH-Wert.

Biopharmazeutische Produktionsprozesse erfordern verschiedene orthogonale Schritte zur Virenreduktion. Ein häufig eingesetztes Verfahren zur Inaktivierung von (umhüllten) Viren ist der Kontakt mit einem sauren Medium.

Virusinaktivierung bei niedrigem pH-Wert im Batch-Modus ist in der biopharmazeutischen Produktion von Wirkstoffen, z. B. Antikörpern, bekannt und vielfach angewendet (Sofer 2003, Virus Inactivation in the 1990s - and into the 21st Century. Part 4. BioPharm International). Hierbei wird das zu inaktivierende Gut, eine Flüssigkeit, die aktive Viren potentiell enthält, in einen geeigneten Behälter eingebracht, mit Hilfe einer sauren Lösung zu einem pH-Wert $\leq$ 4 eingestellt, wenn nötig homogenisiert, und über die geforderte Zeit ruhen gelassen. Die Inaktivierung der Viren geschieht durch den Kontakt der Viren mit der sauren Lösung über eine bestimmte produkt- und prozessabhängige Zeit. Der gesamte Inhalt des Bags erfährt somit die Inaktivierung mit nahezu identischer Verweilzeit und folglich ist die geleistete Virenreduktion in jedem Fluidelement des Behälters ebenfalls nahezu gleich.

Soll nun ein Prozess zur Produktion biopharmazeutischer und biologischer Produkte insbesondere pharmazeutischer Antikörper im kontinuierlichen Betriebsmodus gefahren werden, so müsste die benötigte Haltezeit für die Virusinaktivierung realisiert werden. Eine kontinuierliche Virusinaktivierung bedeutet im Sinne der Anmeldung, dass die Zufuhr von Feedstrom in das Virusinaktivierungsmodul und die Abfuhr des Produktstroms aus dem Virusinaktivierungsmodul pausenlos erfolgen. Der kontinuierliche Betrieb einer Produktionsanlage, umfassend mindestens ein Bioreaktor, bedeutet im Sinne der Anmeldung, dass die Zufuhr von Feedstrom in den Bioreaktor und die Abfuhr des Produktstroms aus der Produktionsanlage pausenlos erfolgen, wobei manche Verfahrensschritte semi-kontinuierlich arbeiten können.

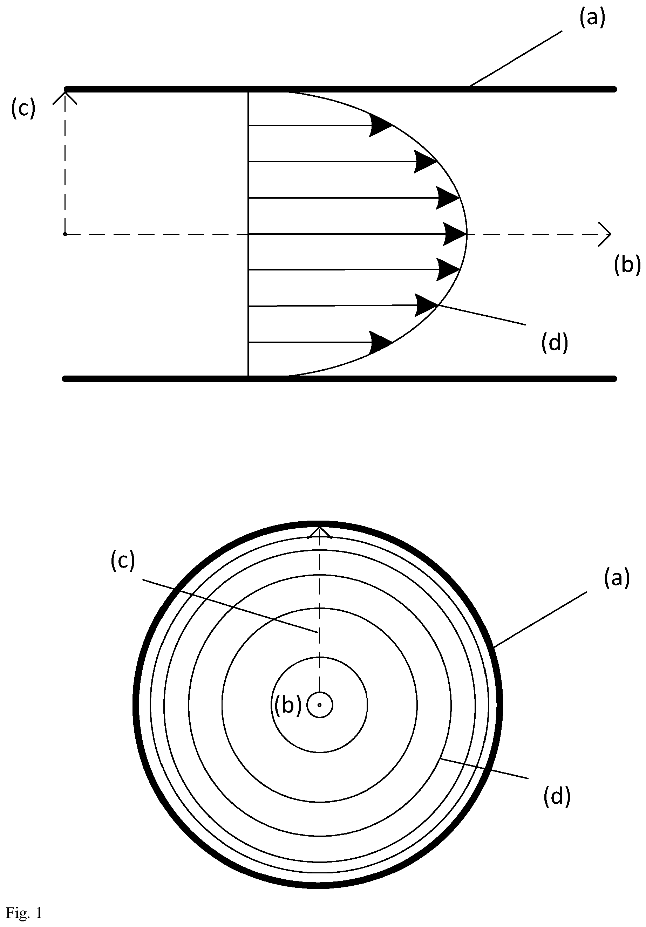

Die benötigte Haltezeit (=Verweilzeit) für die Virusinaktivierung könnte in einer Verweilschleife realisiert werden. Problematisch kann dabei die laminare Durchströmung in der Verweilzeitschleife werden. In einer laminaren Rohrströmung bildet sich ein parabolisches Geschwindigkeitsprofil aus, wodurch es zu einer breiten Verweilzeitverteilung kommt (Fig. 1). Da die Maximalgeschwindigkeit im Zentrum der Rohrströmung das Doppelte der mittleren Geschwindigkeit beträgt, an den Rohrwänden jedoch die Geschwindigkeit gleich Null ist (Haftbedingung), tritt in diesen Fällen eine sehr breite Verweilzeitverteilung auf. Die so erhaltenen Verweilzeiten liegen zwischen der halben durchschnittlichen Verweilzeit (hervorgerufen durch die schnell strömenden Fluidelemente in der Rohrmitte) und einer unendlich langen Verweilzeit (hervorgerufen durch die anhaftenden Fluidelemente in Wandnähe). Da zum einen zur effektiven Inaktivierung der Viren eine Mindestverweilzeit erforderlich ist, zum anderen aber lange Verweilzeiten bei niedrigem pH-Wert das Produkt (wie z. B. ein Protein) schädigen könnten, ist die Realisierung einer engen Verweilzeitverteilung im kontinuierlichen Betrieb unabdingbar. Ein Wechsel von der laminaren Strömungssituation in eine turbulente Kolbenströmung mit einheitlicher Verweilzeit stellt in diesem Fall keine akzeptable Altenative dar. Turbulente Strömungen setzen hohe Fließgeschwindigkeiten voraus. Sollen nun die für Virusinaktivierungen bei niedrigem pH-Wert üblichen langen Verweilzeiten (beispielsweise 60-120 min) realisiert werden, entstehen unvorteilhaft große Anlagen.

Eine Möglichkeit eine kontinuierliche Virusinaktivierung durchzuführen, ist die Bestrahlung mit UV-C Licht: WO2002038191 , EP1339643B1, EP1464342B1, EP1914202A1 und EP1916224A1 beschreiben die Verwendung einer helixförmigen Verweilschleife in der das zu inaktivierende Gut mit UV-C Licht bestrahlt und die vorhandenen Viren in der Folge inaktiviert werden. Durchströmt ein Fluid ein helikal gewickeltes Rohr, so wirkt die Zentrifugalkraft auf das Fluid. Durch diese Zentrifugalkräfte werden Sekundärströmungen (sog. Dean-Wirbel) induziert, was zu einer verbesserten radialen Durchmischung und somit homogeneren Bestrahlung des zu inaktivierenden Guts führt. Die in den genannten Quellen verwendete Helixstruktur ist eine gerade Helixwicklung ohne Richtungsänderungen der Achse der Helix. Für die Anwendung einer kontinuierlichen Virusinaktivierung bei niedrigem pH-Wert ist die Verwendung einer geraden Helix-Struktur, wie sie bei der UV-C Bestrahlung verwendet wird, nicht praktikabel, da die Verweilzeitverteilung zwar enger als beim laminar durchströmten geraden Rohr ist, jedoch immer noch zu breit ausfällt. Bedingt durch die immer noch vergleichsweise breite Verweilzeitverteilung würde auch diese Geometrie für eine pH-Virusinaktivierung weiterhin eine große Anlage erfordern.

Da zum einen jedes Fluidelement eine geforderte Mindestverweilzeit erreichen muss und zum anderen das Produkt insbesondere ein Proteinprodukt bei niedrigem pH-Wert geschädigt werden kann, ist es für eine pH-basierte Virusinaktivierung erforderlich möglichst enge Verweilzeitverteilungen zu erzeugen.

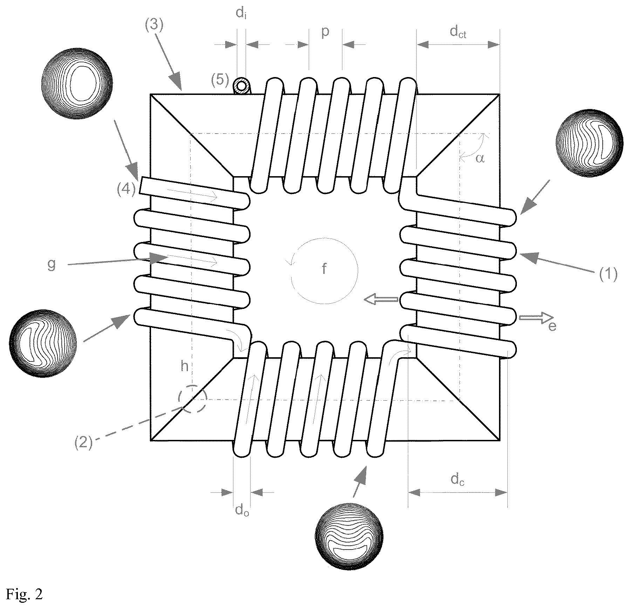

Nigam et al. [US7337835B2, AIChE Journal (1984), Vol. 30, No. 3, S. 363-368, Chem. Eng. Comm. (1983) 23, 4-6, S. 277-289] lehren, dass bei der Durchströmung von Helixwicklungen die Biegung der Achse der Helixwicklungen eine Änderung der Wirkrichtung der Normalen der Zentrifugalkraft auf das Fluid bewirkt.. Die engsten Verweilzeitverteilungen können laut Nigam et al. ab einem Wert für die Dean-Zahl von $Dn \geq 3$ erreicht werden, wohingegen für $Dn < 3$ eine Verbreiterung der Verweilzeitverteilung zu beobachten ist. Diese Technik wurde von den Autoren als "Coiled Flow Inverter" (CFI) bezeichnet. Das Prinzip des CFI wird auf **Fig. 2** dargestellt. Die durch die helikale Rohrgeometrie hervorgerufene Zentrifugalkraft erzeugt Sekundärströmungen womit auch bei laminarer Durchströmung enge Verweilzeitverteilungen in Wärmeübertragern realisiert werden. Nigam et al. lehren, dass durch die Implementierung von 90°-Bends erheblich engere Verweilzeitverteilungen erzeugt werden als in der geraden Helix oder dem laminar durchströmten geraden Rohr. Nigam et al. lehren weiter, dass die Verweilzeitverteilung umso enger ausfällt, je mehr Bends verwendet werden. Mit zunehmender Anzahl von Bends findet eine Annäherung an die Verweilzeitverteilung eines turbulent durchströmten Strömungsrohrs mit Kolbenströmungsprofil statt.

EP0378716A1 offenbart einen UV-Sterilisator mit helikalen Fluidkanälen.

Die Anwendbarkeit dieser Rohrgeometrie für Verfahren, die gleichzeitig eine lange Verweilzeit und eine enge Verweilzeitverteilung erfordern, wie z. B. die Virusinaktivierung bei niedrigen pH-Wert, wird nicht untersucht oder erwähnt. Eine lange Verweilzeit ist für die Bereitstellung eines Wärmeübertragers irrelevant.

Daher war es die Aufgabe der vorliegenden Erfindung, eine neue, einfache und preiswerte Lösung zur Verfügung zu stellen, welche die benötigte Verweilzeit in einer kontinuierlich durchströmten Verweilzeitschleife zur kontinuierlichen Virusinaktivierung bei niedrigem pH-Wert bei enger Verweilzeitverteilung ermöglicht.

Die Erfindung löst diese Aufgabe durch ein Verfahren nach Anspruch 1.

Bevorzugt ist das Rohr oder der Schlauch 1 helikal um die Windungsachse h gewickelt. Üblicherweise ist der Querschnitt der Windungsachse rund.

Bespiel für eine gekrümmte Konfiguration ist aus EP094443181B1 bekannt, insbesondere in Fig. 5 bis 11, die hiermit mit ihrer Beschreibung per Referenz integriert sind.

Das erfindungsgemäße Verfahren kann ein Haltegestell 6 umfassen, welches ein oder mehrere Rahmen 3 trägt. Alternativ bildet das Haltegestell die Windungsachse. Die Rahmen und/oder das Haltegestell können hohl oder gefüllt sein. Verfügt der Schlauch bzw. das Rohr 1 über eine hohe Festigkeit und Steifigkeit, ist auch eine selbstragende Struktur möglich.

Zur Dimensionierung der Anlage wird die Lehre von Nigam et al. per Referenz integriert US7337835B2, AIChE Journal (1984), Vol. 30, No. 3, S. 363-368, Chem. Eng. Comm. (1983) 23, 4-6, S. 277-289. Insbesondere lehrt Nigam et al. dass die Verstärkung der Durchmischung in radialer Richtung, woraus eine Verengung der Verweilzeitverteilung resultiert, bei einem Winkel von $\alpha$ von 45° bis 180 ° bereits eintritt, bevorzugt aber ein Bereich von 40° bis 120°, und besonders 90°. **Fig. 2** stellt das Prinzip des CFI nach Nigam et al. und dessen Designparameter für den besonderen Fall $\alpha$=90° dar. Man erkennt wie sich das Strömungsprofil in Abhängigkeit der Richtung der Helixwicklung verändert.

Als Designparameter sind zu nennen:

- Schlauchinnendurchmesser $d_i$
- Pitch Distanz $p$
- Coil Tube Durchmesser $d_{ct}$
- Coil Durchmesser $d_c$
- Anzahl der Windungen pro Arm n (ein Arm ist der Bereich gerader Helixwicklung zwischen zwei aufeinander folgender Bends)
- Winkel $\alpha$ der Bends
- Anzahl der Bends (im dargestellten Rahmen 4 Bends pro Rahmen)

Die das System beschreibenden dimensionslosen Kennzahlen sind die Reynolds-Zahl *Re*, die Dean-Zahl *Dn* und der Torsion Parameter *T*.

Die Reynolds-Zahl *Re* berechnet sich nach:

$$Re = \frac{\rho \cdot \bar{v} \cdot d_i}{\eta}$$

Mit der Dichte des Fluids $\rho$, der mittleren Strömungsgeschwindigkeit $\bar{v}$ und der dynamischen Viskosität $\eta$.

Die Dean-Zahl *Dn* berechnet sich nach:

$$Dn = Re \cdot \sqrt{d_i/d_c}$$

Der Torsion Parameter *T* berechnet sich nach:

$$T = \frac{\pi \cdot r_c \cdot Re}{p}$$

Nigam et al. lehrt, dass sich die besten Ergebnisse erzielen lassen, wenn die Pitch Distanz minimiert wird, der Winkel $\alpha$=90° beträgt, die Dean-Zahl mindestens 3 beträgt und der Torsion Parameter $\geq$ 500 ist. Damit sich stabile Sekundärströmungen in der Helix ausbilden können, sollten darüber hinaus pro Arm mindestens zwei Windungen vollständig ausgeführt werden. [AIChE Journal (1984), Vol. 30, No. 3, S. 363-368] und [Chem. Eng. Comm. (1983) 23, 4-6, S. 277-289]

Die Dimensionierung der erfindungsgemäßen Vorrichtung erfolgt üblicherweise wie folgt:

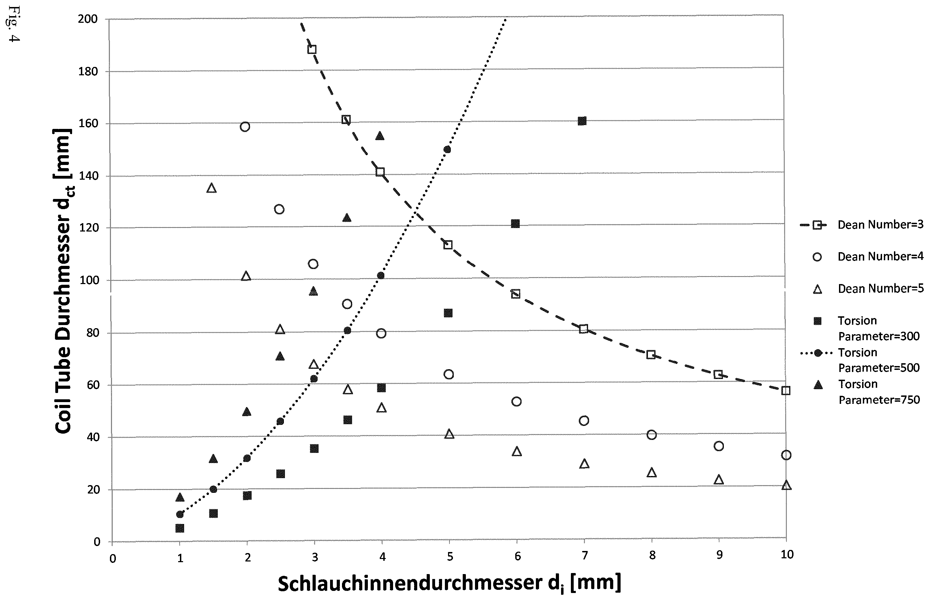

- In einem ersten Schritt wird der gewünschte Volumenstrom festgelegt.
- Auf dieser Basis werden mögliche Dimensionen mit den o. g. Formeln berechnet unter der Bedingung, dass die Dean-Zahl bevorzugt $\geq$ 3 ist und der Torsion Parameter bevorzugt $\geq$ 500 ist.
- Die erzeugten Kurven sind auf **Fig. 4** exemplarisch für einen Volumenstrom von 3 mL/min dargestellt. Die geeigneten Dimensionen für die Vorrichtung sind gemäß Nigam et al. links von der vorteilhaften Kurve Torsion Parameter = 500 und links der vorteilhaften Kurve für einen Dean-Zahl = 3 zu finden. Die sinnvollsten Schlauchinnendurchmesser und Coil Tube Durchmesser werden insbesondere an den Platzbedarf optimiert ausgewählt. Hierbei ist der Schlauchinnendurchmesser so zu wählen, dass die geforderte Mindestverweilzeit erreicht wird. Der Coil Tube Durchmesser sollte daraufhin so klein wie möglich gewählt werden. Grund hierfür ist, dass nach Nigam et al. die Verweilzeitverteilung umso enger wird, je mehr Bends in der Verweilzeitstrecke installiert werden. Für eine definierte Schlauchlänge der Verweilzeitstrecke und für eine feste Anzahl an Windungen pro Helix, können umso mehr Bends installiert werden, je geringer der Coil Tube Durchmesser ist. Näherungsweise lässt sich die Anzahl der Bends $n_{Bend}$ der Verweilzeitstrecke der Länge $L$ berechnen, wobei $n_{Arm,Rahmen}$ die Anzahl der Arme pro Rahmen, $n_{Windung,Arm}$ die Anzahl der Windungen pro Arm, $\pi$ die Zahl pi und $d_c$ der Coil Durchmesser mit $d_c = d_{ct} + d_o$ ist: $d_o$ bezeichnet hier den Außendurchmesser des Schlauchs.

$$n_{Bend} = \frac{L}{n_{Bend,Rahmen} \cdot n_{Windung,Arm} \cdot \pi \cdot d_c} - 1$$

Erfindungsgemäß wurden beispielsweise für eine Testanlage zur kontinuierlichen Virusinaktivierung mit einem Volumenstrom von rund 3 mL/min die o. g. Parameter folgendermaßen ausgewählt. Mit Hilfe der für den jeweiligen Anwendungsfall geforderten Mindestverweilzeit und dem gewählten Schlauchinnendurchmesser ergibt sich die benötigte Schlauchlänge. Im nächsten Schritt ist der Coil Tube Durchmesser so zu wählen, dass sowohl Dean-Zahl $\geq$ 0 als auch Torsion Parameter $\geq$ 0, bevorzugt dass sowohl Dean-Zahl $\geq$ 2, bevorzugt $\geq$ 3, als auch Torsion Parameter $\geq$ 300, bevorzugt, $\geq$ 500, erfüllt ist. Siehe hierzu **Fig. 4.**

Der innere Durchmesser $d_i$ des Rohres oder Schlauchs 1 beträgt üblicherweise von 1 bis 30 mm, bevorzugt zwischen 3 und 6 mm. In der Testanlage wurden beispielsweise ein kommerzielles Rohr/ kommerzieller Schlauch von einem Innendurchmesser von 3 mm verwendet. Darauf folgend wurde der für diesen Fall minimal mögliche Coil Tube Durchmesser gewählt. Je kleiner der Coil Tube Durchmesser gewählt wird, umso mehr Bends können für eine bestehende Schlauchlänge realisiert werden. Da eine steigende Anzahl von Bends die Verweilzeitverteilung verengt, sollte diese Zahl stets möglichst groß gewählt werden.

Die Gesamtlänge L und innerer Durchmesser der Rohre/Schlauche 1 werden an die Dimensionen der Gesamtanlage/Durchflussrate der Anlage so angepasst, dass die im jeweiligen Anwendungsfall geforderten Verweilzeiten eingehalten werden.

Für eine Anlage der o. g. Größe weist das Rohr oder der Schlauch 1 üblicherweise eine Gesamtlänge L von 1 bis 200 m, bevorzugt von 50 bis 100 m auf.

Die Anzahl der Windungen n zwischen zwei Richtungsänderungen und/oder Knicken 2 beträgt üblicherweise mindestens 2 bis 20, bevorzugt 5 bis 15, besonders bevorzugt 10, wobei die Anzahl der Windungen so ausgewählt wird, dass die Unit ein möglichst kleines Volumen beansprucht.

Bildet das Haltegestell die Windungsachse oder ist der Schlauch bzw. das Rohr 1 selbsttragend, weist die Windungsachse üblicherweise 2 bis n Richtungsumkehrungen und/oder Knicke 2 auf, wobei n beliebig sein kann. Die Zahl n wird hierbei so ausgewählt, dass die Gesamtlänge L der Rohre/Schlauche 1 um die Unit gewickelt ist und ein möglichst kleines Volumen beansprucht.

Werden Rahmen als Windungsachse verwendet, weist jeder Rahmen 3 üblicherweise 2 bis 6 Richtungsumkehrung und/oder Knicke 2 auf. Bevorzugt sind quadratische Rahmen (90 ° Knicke) wie in in **Fig. 2** dargestellt, ohne sich darauf zu begrenzen. Ein oder mehrere Rahmen werden üblicherweise übereinander an das Gestell 6 befestigt, bis die Gesamtlänge L des Rohres/Schlauchs 1 um die Unit gewickelt ist und ein möglichst kleines Volumen beansprucht.

Die so erzielte enge Verweilzeitverteilung ermöglicht die geforderte Virenabreicherung bei einer bestimmten produkt- und prozessabhängigen minimalen Verweilzeit, ohne die maximale, ebenfalls produkt- und prozessabhängige Verweilzeit zu erreichen, die zu Produktschädigung führen würde (typischerweise von 30 min für pH-empfindliche

EP 3 116 552 B1

Produkte bis 120 min für weniger empfindliche Produkte). Die erforderliche Verweilzeit sowie die Maximalverweilzeit sind produktabhängig und werden typischerweise experimentell ermittelt. Die Maximalverweilzeit wird so optimiert, dass das Produkt minimal beschädigt wird, um den Bedarf an nachgeschalteten Reinigungschritten so gering wie möglich zu halten. Die Verweilzeitverteilung nähert sich der mittleren Verweilzeit des idealen Strömungsrohrreaktors an. Hiermit kann eine effektive kontinuierlich betriebene Virusinaktivierung bei niedrigem pH-Wert sichergestellt werden, deren Ergebnisse bzgl. Virenabreicherung und Produktqualität vergleichbar mit der Virusinaktivierung in einem Batch-Verfahren wären.

Insbesondere bei der Nutzung von Rahmen auf einem Gestell ist eine einfache, skalierbare und kostengünstige Fertigung der Vorrichtung (auch für den Einweggebrauch) möglich. Der Schlauch/ das Rohr wird lediglich in der geforderten Art und Weise um die Rahmen gewickelt nachdem oder bevor der Schlauch/ das Rohr sterilisiert wurde. Nach einem Einsatz der Einheit im Prozess, kann der Schlauch/ das Rohr vom Rahmen gelöst und entsorgt bzw. gereinigt werden (sofern Mehrfachverwendung gewünscht ist). Die Winkel der Bends sollten definierte Werte haben. Die Position des Schläuchs/ Rohres sollte ebenfalls definiert sein, z. B. für ein einfaches und reproduzierbares Wickeln durch eingefräste Führungsnuten in die Rahmen. Für den Betrieb in einer Anlage wird somit sichergestellt, dass die Anlage in jedem Produktionslauf die gleiche Effektivität besitzt.

Weiterhin kann die Vorrichtung sterilisierbar sein, bevorzugt autoklavierbar oder gammabestrahlbar sein. Für diese Eigenschaft wird bevorzugt ein Schlauch verwendet, der den einschlägigien Qualitätsansprüchen, z. B. medizinische Qualität (USP Class VI) entspricht. Bevorzugt kann die erfindungsgemäße Vorrichtung autoklaviert oder gammasterilisiert werden, was einen sterilen Betrieb ermöglicht.

Weiterhin wird ein Verfahren zur kontinuierlichen Virusinaktivierung eines Produktstroms bereitgestellt, umfassend die folgenden Schritte:

a) Bereitstellen des zu inaktivierenden Produktstroms,
b) Zufuhr des Produktstroms in den Eintritt 4 eines Rohrs oder Schlauchs 1 mit einem Eintritt 4 und einem Austritt 5, wobei das Rohr oder der Schlauch 1 gekrümmt und/oder helikal mit einer Zahl von n Windungen um eine Windungsachse h gewickelt ist und eine oder mehrere Richtungsänderungen und/oder Knicke 2 der Windungsachse mit einem Winkel $\alpha$ von 45° bis 180° zur Änderung der Wirkrichtung der Normalen der Zentrifugalkraft aufweist, wobei die Vorrichtung durch eine Dean-Zahl $\geq$ 0 und einen Torsion Parameter $\geq$ 0 gekennzeichnet ist,
c) Durchlaufen des Produktstroms durch das Rohr oder den Schlauch 1 unter vireninaktivierenden Bedingungen und
d) Ausströmen aus dem Rohr oder dem Schlauch 1 durch den Austritt 5.

In dem Schritt a) wird ein Produktstrom an Flüssigkeit erzeugt, der sowohl Produkt als auch potentiell zu inaktivierende Viren enthalten kann.

Die Vorrichtung in Schritt b) des erfindungsgemäßen Verfahrens kann außer einer Dean-Zahl $\geq$ 0 auch eine Dean-Zahl $\geq$ 1, bevorzugt $\geq$ 2, bevorzugt $\geq$ 3, weiterhin bevorzugt $\geq$ 4 aufweisen.

Die Vorrichtung in Schritt b) des erfindungsgemäßen Verfahrens kann außer einem Torsion-Parameter von $\geq$ 0 auch einen Torsion-Parameter $\geq$ 100, $\geq$ 200, $\geq$ 300, $\geq$ 400, besonders bevorzugt von $\geq$ 500 aufweisen.

In einer besonders bevorzugten Ausführungsform weist die Vorrichtung in Schritt b) des erfindungsgemäßen Verfahrens eine Dean-Zahl $\geq$ 3 und einen Torsion-Parameter $\geq$ 500 auf.

Als mögliche vireninaktivierende Bedingungen für Schritt c) werden ein pH Wert $\leq$ 4, UV- oder thermische Behandlung genannt.

Bevorzugt wird im Schritt a) der pH-Wert des Produktstroms auf einen Wert $\leq$ 4 eingestellt, sofern der pH-Wert des zu inaktivierenden Guts nicht bereits den geforderten Wert hat. In diesem Fall wird üblicherweise nach Schritt d) der pH-Wert auf $\geq$ 5 mit einer Base eingestellt, um die Virusinaktivierung zu beenden.

Einstellen des pH-Wertes der zu inaktivierenden Lösung auf $\leq$ 4 kann beispielsweise durch Zugabe von HCl-Lösung erfolgen. Die Zugabe erfolgt typischerweise im Vorfeld der erfindungsgemäßen Vorrichtung.

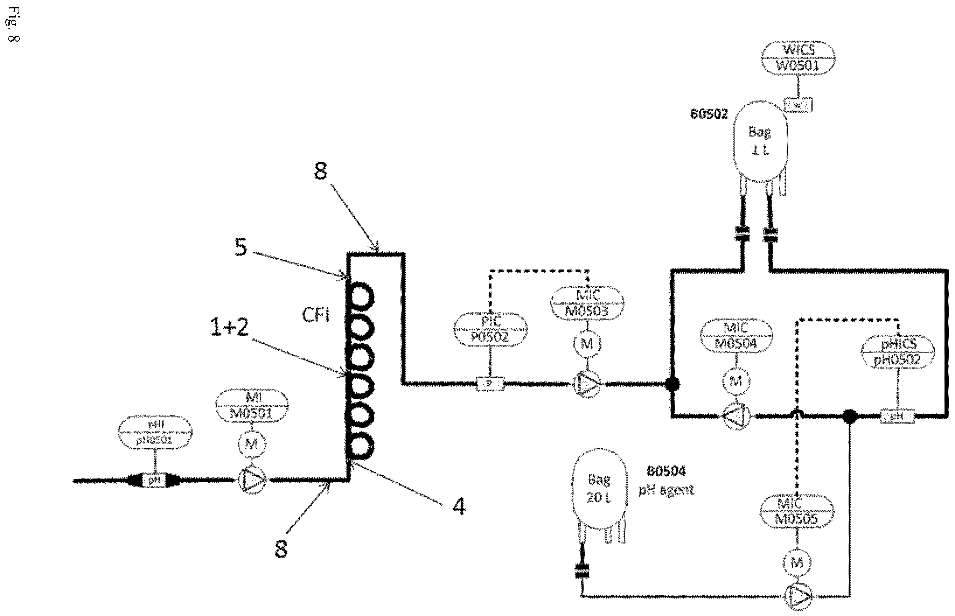

Der pH-Wert des Produktstroms vor Eintritt in die Vorrichtung zur Virusinaktivierung wird durch den Sensor pH0501 erfasst (Fig. 8). Üblicherweise hat dieser pH-Sensor keine Regelaufgaben. Das Aufzeichnen des pH-Signals dient lediglich zur Prozessüberwachung.

Erfordert der Produktionsprozess eine oder mehrere Einstellungen des pH-Wertes wird die Vorrichtung zur Virusinaktivierung an eine Unit zur Einstellung des pH-Wertes angeschlossen. Üblicherweise werden zwei Units zur Einstellung des pH-Wertes verwendet, die erste vor der Inaktivierung zur Einstellung des Produktstroms auf einen pH-Wert $\leq$ 4 (Schritt b), eine weitere nach der Inaktivierung zur Neutralisierung des Produktstroms (Schritt d).

In Schritt c) erfolgt die gewünschte Kontaktzeit (=Verweilzeit) zwischen saurer Lösung und eventuell vorhandenen Viren.

Als Base kann in Schritt d) beispielsweise Natriumhydroxid-Lösung (NaOH) verwendet werden.

Das Verfahren kann als Batch-Operation und als kontinuierliches Produktionsverfahren durchgeführt werden und somit in einen Batch-Prozess und in einen kontinuierlichen Prozess integriert werden.

5

Wird die Vorrichtung zur Virusinaktivierung in einem kontinuierlichen Produktionsprozess integriert, so wird eine oder mehrere Units zur Einstellung des pH-Wertes bevorzugt, in der der Produktstrom eine Rezirkulationsschleife durchströmt. **Fig. 8** stellt die Virusinaktivierung und eine anschließende Neutralisierung exemplarisch dar, ohne sich darauf zu begrenzen. M0503 fördert den Produktstrom in den Bag B0502 wo der pH-Wert nach Verlassen der Virusinaktivierung auf einen Wert $\geq 5$ eingestellt wird. Den Inhalt des Bags B0502 fördert die Rezirkulationspumpe M0504 durch die Rezirkulationsschleife in der der pH-Sensor pH0502 den pH-Wert des Produktstroms misst. Strömungstechnisch hinter dem Sensor wird das Stellmittel zur Anpassung des pH-Werts zudosiert. Die Pumpe des Stellmittels ist M0505 und wird über den zugehörigen Sensor pH0502 geregelt.

In dem erfindungsgemäßen Verfahren ist der zu inaktivierende Produktstrom üblicherweise eine Lösung aus einem Bioreaktor, insbesondere eine Protein- oder Peptidlösung und ganz besonders eine Antikörperlösung.

Der technische Vorteil der erfindungsgemäßen kontinuierlichen Virusinaktivierung gegenüber der im Stand der Technik üblichen Virusinaktivierung im Batch-Modus besteht in der Integrierbarkeit in einen kontinuierlichen Aufarbeitungsprozess, auch "Downstream Processing" genannt ohne die Prozessfahrweise ändern zu müssen. Hierbei kommt es nicht zu einem Wechsel in der Prozessführung von Batch auf kontinuierlich und wieder zurück, sondern der gesamte "Downstream Processing" ggf. der gesamte Produktionsprozess (Upstream und Downstream) kann kontinuierlich durchfahren werden.

Die vorliegende Erfindung einschließlich bevorzugter Ausführungsformen wird in Verbindung mit den nachfolgenden Zeichnungen und Beispielen erläutert, ohne hierauf beschränkt zu sein. Die Ausführungsformen können beliebig miteinander kombiniert werden, sofern sich nicht eindeutig das Gegenteil aus dem Kontext ergibt.

Die verwendeten Bezugszeichen sind:

1 = gekrümmtes und/oder helixförmig gewickeltes Rohr oder Schlauch
2 = Richtungsumkehrungen und/oder Knicke 2 der Windungsachse h mit einem Winkel $\alpha$ von 45° bis 180°
3 = Rahmen
4 = Eintritt
5 = Austritt
6 = Haltegestell
7 = Fuß
8 = Produktstromleitung

**Fig. 1** zeigt ein Parabolisches Strömungsprofil des laminar durchströmten Rohres (oben: Längsschnitt des Rohres). Linien gleicher Geschwindigkeit in Strömungsrichtung im laminar durchströmten Rohr (unten: Querschnitt des Rohres).

a = Rohrwand
b = Axialachse des Rohres in Strömungsrichtung
c = Radialachse
d = Linien gleicher Strömungsgeschwindigkeit in Strömungsrichtung

**Fig. 2** zeigt Prinzip und Designparameter des CFI mit Darstellung des Strömungsprofils. Dargestellt sind Linien gleicher Geschwindigkeit in Strömungsrichtung. Strömungsprofile wurden entnommen aus: Ind. Eng. Chem. Res. (2008), 47, 10, S. 3630-3638

- Schlauchinnendurchmesser $d_i$
- Schlauchaußendurchmesser $d_o$
- Pitch Distanz $p$
- Coil Tube Durchmesser $d_{ct}$
- Coil Durchmesser $d_c$
- Winkel $\alpha$ der Bends

e = Wirkrichtung der Zentrifugalkraft
f = Durchströmrichtung der Helixwicklungen
g = Strömungsrichtung des Fluids in der Helixwicklung
h = Windungsachse/Helixachse

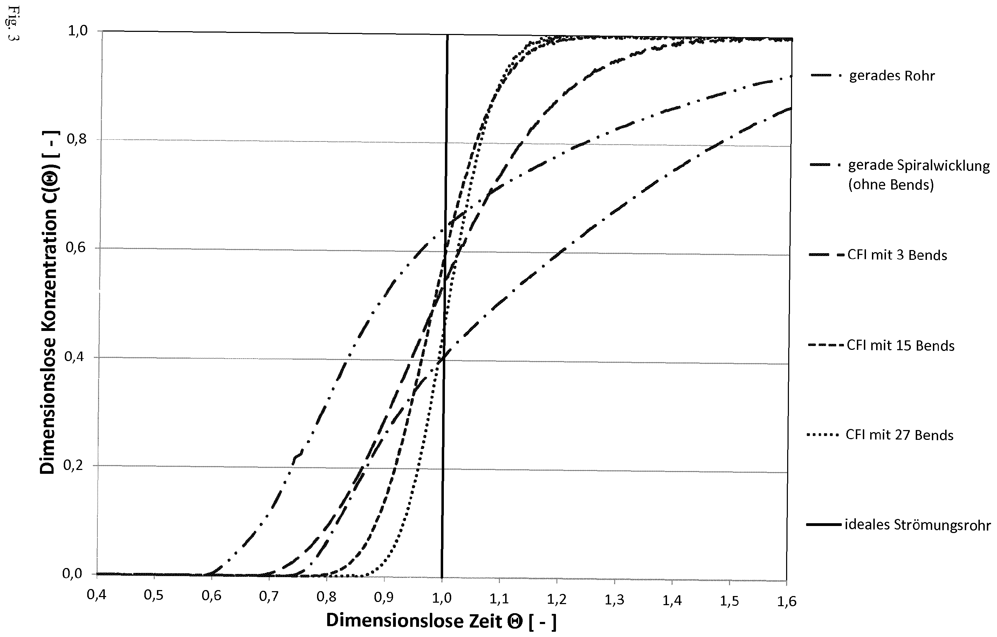

**Fig. 3** zeigt die Ergebnisse der Untersuchung des Verweilzeitverhaltens der Vorrichtung zur kontinuierlichen Virusinaktivierung mit verschiedenen Anzahlen von Bends bei einem Volumenstrom von 3 mL/min im Vergleich zu einem geraden laminar durchströmten Rohr und einem idealen Strömungsrohr. (Bend = 90°-Knick zur Änderung Normalen der Wirkrichtung der Zentrifugalkraft auf die Strömung. Die dimensionslose Konzentration von 1 entspricht einer Konzentration an Vitamin B12 von 0,25 g/L)

**Fig. 4** zeigt das Diagramm zur Auslegung des CFI bei einem Volumenstrom von 3 mL/min

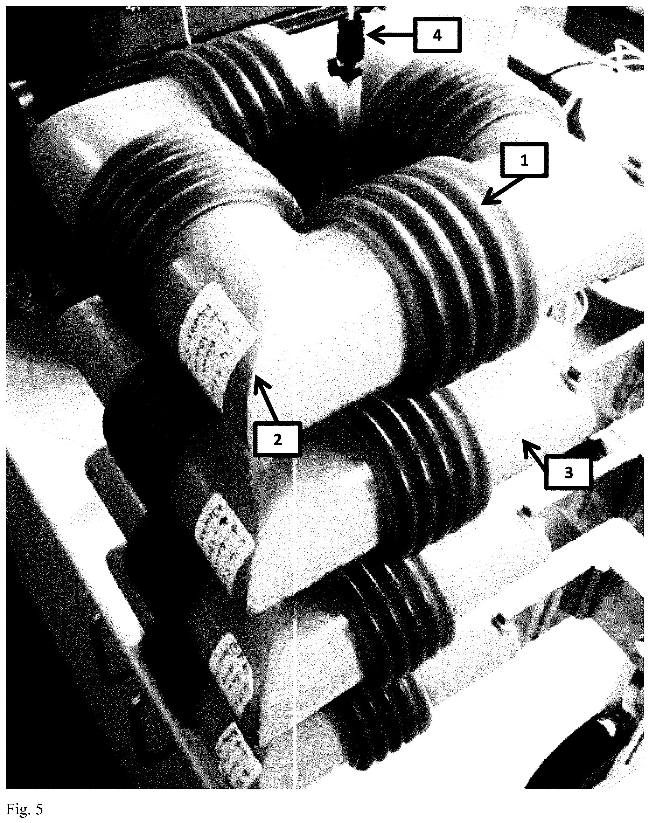

**Fig. 5** zeigt eine Aufnahme der Vorrichtung wie sie in den Experimenten zur Verweilzeitmessung verwendet wurde (4 Rahmen).

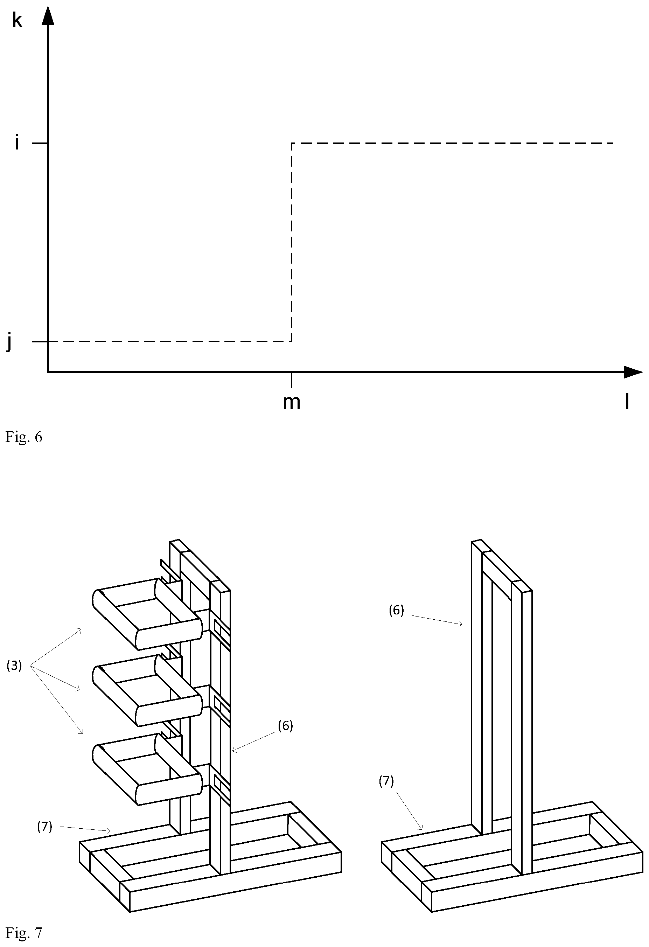

**Fig. 6** zeigt die in Beispiel 1 aufgegebene Stufenfunktion der Tracer-Lösung am Eintritt in die Vorrichtung zur Virusinaktivierung

i = Zeitachse

j = Achse des UV-Signals

k = Zeitpunkt der Aufgabe der Tracer-Substanz am Eintritt des CFI

**Fig. 7** zeigt die Zeichnung der Vorrichtung zur kontinuierlichen Virusinaktivierung nach dem Vorbild des CFI. Links: Haltegestell mit montierten Rahmen. Rechts: Haltegestell ohne montierte Rahmen.

**Fig. 8** Fließbild der Virusinaktivierung mit anschließender Anpassung des pH-Wertes, wobei das helixförmig gewickelte Rohr 1 und dessen Richtungsumkehrungen und/oder Knicke 2 lediglich schematisch dargestellt sind.

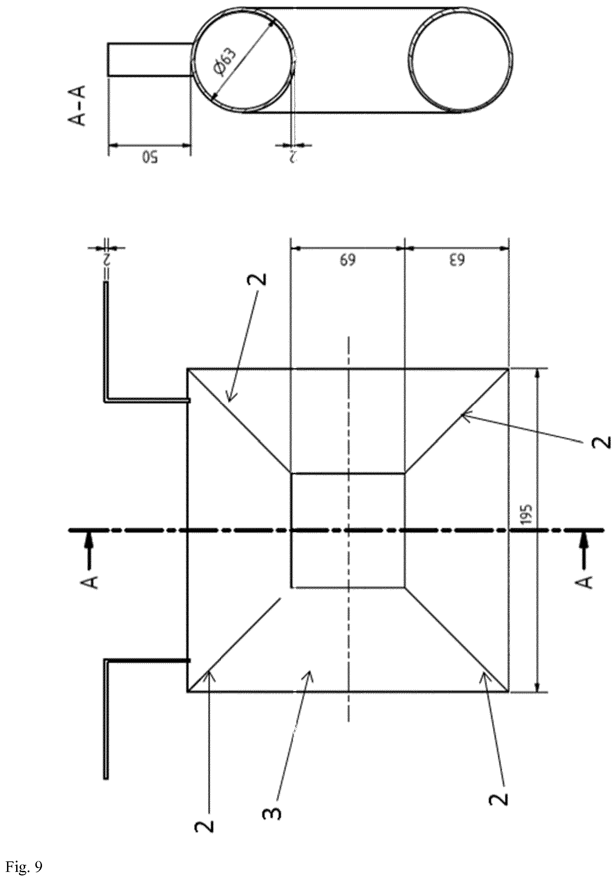

**Fig. 9** Konstruktionszeichnung eines Rahmens aus den experimentellen Untersuchungen.

### Beispiel 1:

Die Auslegung der Rahmen wurde nach **Fig. 4** durchgeführt. Dieses Diagramm wurde für eine Flussrate von 3 mL/min erstellt und zeigt in welchem Bereich sich die Gestaltungsparameter Schlauchinnendurchmesser und Coil Tube Durchmesser variieren lassen um die geforderten Bedingungen Dean-Zahl ≥ 3 und Torsion Parameter ≥ 500 bei der Konstruktion der Rahmen einzuhalten.

Für die experimentellen Untersuchungen wurde ein Schlauchinnendurchmesser von 3 mm gewählt. Der Coil Tube Durchmesser wurde daraufhin zu 63 mm gewählt, sodass die aufgewickelte Schlauchlänge pro Rahmen minimiert werden konnte.

Die Maße des in den experimentellen Untersuchungen verwendeten Aufbaus, deren Ergebnisse in **Fig. 3** dargestellt sind, lauten:

Rahmendurchmesser (Coil Tube Durchmesser) 63 mm; äußere Kantenlänge der Rahmen 195 mm. Schlauchinnendurchmesser 3 mm; Schlauchaußendurchmesser 5 mm. Die Rahmen wurden entsprechend nach **Fig. 9** gefertigt.

Pro Arm wurden stets 11 Wicklungen mit minimaler Pitch Distanz ausgeführt, sodass auf einem Rahmen 9,5 m Schlauch aufgewickelt wurden. Minimale Pitch Distanz bedeutet, dass der Schlauch in der Helix aneinander liegt. Für den Fall "3 Bends" wurde ein Rahmen verwendet. Folglich wurden für dieses Experiment 9,5 m Schlauch aufgewickelt. Für den Fall "15 Bends" wurden vier Rahmen verwendet. Auf vier Rahmen wurden insgesamt 38 m Schlauch gewickelt. Für den Fall "27 Bends" wurden sieben Rahmen verwendet. Auf sieben Rahmen wurden insgesamt 66,5 m Schlauch gewickelt. Die pro Rahmen verwendete Schlauchlänge verhält sich unter der Annahme konstanter Windungszahlen pro Arm proportional zum Coil Tube Durchmesser. Der Schlauchaußendurchmesser betrug beim verwendeten Schlauch 5 mm.

**Fig. 5** zeigt die Anordnung der Rahmen und Schlauchwicklungen wie sie in den Versuchen zur Verweilzeitmessung angeordnet wurden. Für diese Abbildung wurde allerdings aus Gründen der Anschaulichkeit ein größerer Schlauchdurchmesser (Schlauchinnendurchmesser 6 mm) gewählt. Folglich waren auch nicht die in den Experimenten verwendeten 11 Wicklungen pro Arm möglich. Im experimentellen Aufbau wurde zunächst jeder Rahmen separat mit dem im jeweiligen Experiment verwendeten Schlauch wie in **Fig. 2** und **Fig. 5** dargestellt umwickelt. Anschließend wurden die beschlauchten Rahmen an einem Haltegestell übereinander angeordnet. Hierbei wurde der Ausgang des oberen Rahmens mit dem Eingang des darunter liegenden Rahmens verbunden, sodass die Schlauchwicklung der Rahmen von oben nach unten durchlaufen wurde. Alternativ kann man auch von unten nach oben oder in der Horizontalen durchströmen.

Die Versuche zur Verweilzeitmessung in der Vorrichtung zur kontinuierlichen Virusinaktivierung (CFI) wurden mit Hilfe einer UV-Messung am Austritt des Systems durchgeführt. Die Flussraten betrugen stets 3 mL/min, der Innendurchmesser der verwendeten Silikonschläuche betrug 3 mm, der Außendurchmesser der Schläuche betrug 5 mm. Die Außendurchmesser der von den Schläuchen umwickelten Rahmen betrugen 63 mm (Coil Tube Durchmesser). Als Tracer-Substanz wurde eine Vitamin B12 Lösung mit einer Konzentration von 0,25 g/L verwendet, da Vitamin B12 UV-Licht bei einer Wellenlänge von 280 nm absorbiert und sich somit als Indikator eignet.

Zunächst wurde der CFI mit destilliertem Wasser gespült. Zum Zeitpunkt k wurde am Eintritt der Virusinaktivierung auf die Tracer-Lösung umgeschaltet und die Aufzeichnung des Messsignals des UV-Sensors gestartet (siehe **Fig. 6).** Es wurde folglich eine Stufenfunktion der Tracer-Lösung auf das System aufgegeben. Wenn das UV-Signal am Austritt des Systems dem UV-Signal der Tracer-Lösung entsprach, konnten die Versuche beendet werden, da das System ab diesem Zeitpunkt komplett mit Tracer-Lösung gefüllt und somit die Antwort des Systems auf die Stufenfunktion vollständig aufgezeichnet war.

Damit die verschiedenen aufgenommenen Verweilzeitkurven miteinander verglichen werden konnten, wurde auf dimensionslose Größen normiert. Die Zeit wurde auf die mittlere Verweilzeit $\tau$ normiert.

$$\tau = \frac{V}{\dot{V}}$$

Wobei $V$ das Holdup Volumen der Verweilzeitstrecke ist und $\dot{V}$ der Volumenstrom.

Die dimensionslose Konzentration ergab sich, indem das gemessene UV-Signal auf das maximal aufgenommene UV-Signal (bei einer Vitamin B12 Konzentration von 0,25 mg/L) normiert wurde. Eine Vitamin B12 Konzentration von 0,25 mg/L ergibt folglich eine dimensionslose Konzentration von 1. Das UV-Signal von destilliertem Wasser führt zu einer dimensionslosen Konzentration von 0.

Die Ergebnisse der Messungen sind in **Fig. 3** dargestellt. Die Verweilzeitverteilung des geraden laminar durchströmten Rohres wurde analytisch nach folgender Gleichung bestimmt. Es bedeuten $F(\theta)$ die dimensionslose Konzentration und $\theta$ die dimensionslose Zeit.

$$F(\theta) = 1 - \frac{1}{4 \cdot \theta^2}$$

Das laminar durchströmte gerade Rohr hat aufgrund seines parabolischen Strömungsprofils eine vergleichsweise breite Verweilzeitverteilung. Im Rohrzentrum strömen die Fluidelemente deutlich schneller als in den wandnahen Bereichen (siehe **Fig. 1).**

Wird der Schlauch hingegen helikal aufgewickelt, bewirkt die Zentrifugalkraft eine Durchmischung des Systems in radialer Richtung. Als Folge dessen bewegen sich langsam strömende Fluidelemente, die näher an der Helixachse sind, nach außen und verdrängen die dort befindlichen Elemente nach innen. Durch die Implementierung von Bends bauen sich die durch die Zentrifugalkraft hervorgerufenen Sekundärströmungen neu aus, was zu einer verbesserten radialen Durchmischung führt. **Fig. 2** zeigt, wie sich das Strömungsprofil nach jedem 90°-Bend um 90° dreht. Wie aus **Fig. 3** zu entnehmen ist, kann durch die Verwendung von Bends eine deutliche Verengung der Verweilzeitverteilung erreicht werden.

Das beste bei den Versuchen erzielte Ergebnis trat bei der Verwendung des CFI mit 27 Bends auf (eine höhere Zahl von Bends wurde nicht untersucht). Es gelang eine Annäherung an die Verweilzeitverteilung des idealen Strömungsrohrs. Eine weitere Verengung der Verweilzeitverteilung durch zusätzliche Bends ist wahrscheinlich.

Die beschriebene Technik ist somit als Verfahren zur kontinuierlichen Virusinaktivierung geeignet.

**Patentansprüche**

1. Verfahren zur kontinuierlichen Virusinaktivierung eines Produktstroms, umfassend die folgenden Schritte:

   a) Bereitstellen des zu inaktivierenden Produktstroms,
   b) Zufuhr des Produktstroms in den Eintritt (4) eines Rohrs oder Schlauchs (1) mit einem Eintritt (4) und einem Austritt (5), wobei das Rohr oder der Schlauch (1) gekrümmt und/oder helikal mit einer Zahl von n Windungen um eine Windungsachse h gewickelt ist und eine oder mehrere Richtungsänderungen und/oder Knicke (2) der Windungsachse mit einem Winkel a von 45° bis 180° zur Änderung der Wirkrichtung der Normalen der Zentrifugalkraft aufweist, wobei das Verfahren durch eine Dean-Zahl $\geq 2$ und einen Torsion-Parameter $\geq 100$ gekennzeichnet ist,
   c) Durchlaufen des Produktstroms durch das Rohr oder den Schlauch (1) unter vireninaktivierenden Bedingungen und
   d) Ausströmen aus dem Rohr oder dem Schlauch (1) durch den Austritt (5).

2. Verfahren nach Anspruch 1, **dadurch gekennzeichnet, dass** die Vorrichtung in Schritt b) durch eine Dean-Zahl $\geq 3$, und einen Torsion-Parameter $\geq 300$, bevorzugt $\geq 500$, gekennzeichnet ist.

3. Verfahren nach Anspruch 1 oder 2, wobei im Schritt a) der pH-Wert des Produktstroms auf einen Wert $\leq 4$ eingestellt wird, sofern der pH-Wert des zu inaktivierenden Guts nicht bereits den geforderten Wert hat.

4. Verfahren nach einem der Ansprüche 1 bis 3, **dadurch gekennzeichnet, dass** die zu inaktivierende Lösung eine Lösung von Makromolekülen, bevorzugt eine Protein- oder Peptidlösung, besonders bevorzugt eine Antikörperlösung ist.

**Claims**

1.  Method for the continuous deactivation of viruses in a product stream, comprising the following steps:

    a) provision of the product stream to be deactivated,
    b) feed of the product stream into the inlet (4) of a tube or hose (1) having an inlet (4) and an outlet (5), where the tube or hose (1) is curved and/or wound helically around a winding axis h with a number of windings n and has one or more changes of direction and/or kinks (2) of the winding axis with an angle a of 45° to 180° for changing the direction of action of the perpendicular of the centrifugal force, where the method is **characterised by** a Dean number $\geq 2$ and a torsion parameter $\geq 100$,
    c) passage of the product stream through the tube or hose (1) under virus-deactivating conditions and
    d) outflow from the tube or hose (1) through the outlet (5).

2.  Method according to Claim 1, **characterised in that** the device in step b) is **characterised by** a Dean number $\geq 3$ and a torsion parameter $\geq 300$, preferably $\geq 500$.

3.  Method according to Claim 1 or 2, where, in step a), the pH of the product stream is adjusted to a value $\leq 4$ if the pH of the material to be deactivated does not already have the required value.

4.  Method according to one of Claims 1 to 3, **characterised in that** the solution to be deactivated is a solution of macromolecules, preferably a protein or peptide solution, particularly preferably an antibody solution.

**Revendications**

1.  Méthode de désactivation continue de virus dans un flux de produit, comprenant les étapes suivantes :

    a) mise à disposition du flux de produit à désactiver,
    b) introduction du flux de produit dans l'entrée (4) d'un tube ou d'un tuyau (1) comportant une entrée (4) et une sortie (5), le tube ou le tuyau (1) étant courbé et/ou enroulé en hélice autour d'un axe d'enroulement h avec un nombre d'enroulements n et présentant un ou plusieurs changements de direction et/ou coudes (2) de l'axe d'enroulement avec un angle a allant de 45° à 180° pour modifier la direction d'action de la perpendiculaire à la force centrifuge, la méthode étant **caractérisée par** un nombre de Dean $\geq 2$ et un paramètre de torsion $\geq 100$,
    c) passage du flux de produit à travers le tube ou le tuyau (1) dans des conditions de désactivation des virus et
    d) écoulement hors du tube ou du tuyau (1) par la sortie (5).

2.  Méthode selon la revendication 1, **caractérisée en ce que** le dispositif de l'étape b) est **caractérisé par** un nombre de Dean $\geq 3$ et un paramètre de torsion $\geq 300$, préférablement $\geq 500$.

3.  Méthode selon la revendication 1 ou 2, dans laquelle, à l'étape a), le pH du flux de produit est ajusté à une valeur $\leq 4$ si le pH de la matière à désactiver n'a pas déjà la valeur requise.

4.  Méthode selon l'une parmi les revendications 1 à 3, **caractérisée en ce que** la solution à désactiver est une solution de macromolécules, de préférence une solution de protéines ou de peptides, particulièrement préférablement une solution d'anticorps.

(a)

(c)

(b)

(d)

(c)

(a)

(b)

(d)

Fig. 1

Fig. 2

Fig. 3

EP 3 116 552 B1

Fig. 4

Fig. 5

Fig. 6

Fig. 7

Fig. 8

Fig. 9

**IN DER BESCHREIBUNG AUFGEFÜHRTE DOKUMENTE**

**In der Beschreibung aufgeführte Patentdokumente**

- WO 2002038191 A
- EP 1339643 B1
- EP 1464342 B1
- EP 1914202 A1
- EP 1916224 A1
- US 7337835 B2
- EP 0378716 A1
- EP 094443181 B1

**In der Beschreibung aufgeführte Nicht-Patentliteratur**

- **SOFER**. Virus Inactivation in the 1990s - and into the 21st Century. *BioPharm International*, 2003
- *AIChE Journal*, 1984, vol. 30 (3), 363-368
- *Chem. Eng. Comm.*, 1983, vol. 23 (4-6), 277-289
- *Ind. Eng. Chem. Res.*, 2008, vol. 47 (10), 3630-3638